# EUROPEAN PATENT APPLICATION

(11) **EP 2 241 312 A1**
(43) Date of publication of application: **20.10.2010**
(21) Application number: 09004572.5
(22) Date of filing: 30.03.2009
(51) Int. Cl.: A61K 31/17, A61P 17/00, A61P 17/02

(54) **Use of compositions comprising urea for treating microbial infections**

(71) Applicant: ISDIN S.A., 08006 Barcelona (ES)
(72) Inventor: Trullas Cabanas, Carlos Ramón, 08232 Viladecavalls (ES); Krutmann, Jean Prof. Dr., 40225 Düsseldorf (DE)
(74) Representative: Kutzenberger, Helga

(57) **Abstract**

The present invention relates to the use of a composition comprising urea for increasing the expression of at least one gene encoding an antimicrobial peptide, preferably a defensin, a cathelicidin such as LL-37 or a psoriasin. Furthermore the invention relates to the use of said compositions for the treatment of microbial infections, preferably microbial infections of the skin.

## Description

The present invention relates to the use of a composition comprising urea for increasing the expression of at least one gene encoding an antimicrobial peptide, preferably a defensin, a cathelicidin such as LL-37 or a psoriasin. Furthermore the invention relates to the use of said compositions for the treatment of microbial infections, preferably microbial infections of the skin.

The immune system protects the body against numerous hazards, in particular infections caused by microorganisms.
It is a highly complex system encompassing numerous specific cell types expressing a multitude of peptides and proteins specific for this system.

The immune system can be subdivided into the innate immune system and the specific or adaptive immune system.

The most prominent proteins of the latter are the antibodies.

Prominent proteins of the innate immune system include e.g. the components of the complement system.

Furthermore the innate immune system includes a group of peptides called "antimicrobial peptides" protecting the body against microorganisms.

These peptides are potent, broad spectrum antibiotics. They have been demonstrated to kill gram-negative and gram-positive bacteria, enveloped viruses, fungi and even transformed or cancerous cells.

Antimicrobial peptides are short peptides, generally between 12 and 50 amino acids. Their ability to associate with membranes is a definitive feature of antimicrobial peptides although membrane permeabilization is not necessary. They have a variety of antimicrobial activities ranging from membrane permeabilization to action on a range of cytoplasmic targets.

The modes of action by which antimicrobial peptides kill bacteria vary and include disrupting membranes, interfering with metabolism, and targeting cytoplasmic components. In many cases the exact mechanism of killing is not known. In contrast to many conventional antibiotics these peptides appear to be bacteriocidal (bacteria killer) instead of bacteriostatic (bacteria growth inhibitor).

In addition to killing bacteria directly they have been demonstrated to have a number of immunomodulatory functions that may be involved in the clearance of infection, including the abililty to alter host gene expression, act as chemokines and/or induce chemokine production, inhibit lipopolysaccharide induced pro-inflammatory cytokine production, promote wound healing, and modulate the responses of dendritic cells and cells of the adaptive immune response.

Examples of antimicrobial peptides include magainins, alamethicin, pexiganan or MSI-78, and other MSI peptides like MSI-843 and MSI-594, polyphemusin, human antimicrobial peptide, protegrins, psoriasins, peptides of the cathelicidin family such as the human LL-37 and defensins.

Defensins are small cysteine-rich cationic proteins found in both vertebrates and invertebrates. They are active against bacteria, fungi and many enveloped and nonenveloped viruses. They consist of 18-45 amino acids including six (in vertebrates) to 8 conserved cysteine residues. Cells of the immune system contain these peptides to assist in killing phagocytized bacteria, for example in neutrophil granulocytes and almost all epithelial cells. Most defensins function by binding to microbial cell membrane, and once embedded, forming pore-like membrane defects that allow efflux of essential ions and nutrients.

The underlying genes responsible for defensin production are highly polymorphic. Some aspects are conserved, however, the hallmarks of a β-defensin are its small size, high density of cationic charge and six-cysteine-residue motif. Generally they are encoded by two-exon genes, where the first exon encodes for a hydrophobic leader sequence and the second for a peptide containing the cysteine motif.

There are three main (known) forms of mammalian defensins, α-defensins, β-defensins and θ-defensins.

The α-defensins are expressed primarily in neutrophils as well as in NK cells and certain T-lymphocyte subsets. Some variants are expressed in Paneth cells of the small intestine, where they may regulate and maintain microbial balance in the intestinal lumen.
The β-defensins are the most widely distributed, being secreted by leukocytes and epithelial cells of many kinds. For example, they can be found on the tongue, skin, cornea, salivary glands, kidneys, esophagus, and respiratory tract. It has been suggested (but also challenged) that some of the pathology of cystic fibrosis arises from the inhibition of β-defensin acitivity on the epithelial surfaces of the luns and trachea due to higher salt content. Different versions of the β-defensins can be found in the human body, e.g. β-Defensin 1, β-Defensin 2 and β-Defensin 3.
The θ-defensins so far have only been identified in primates including humans.

LL-37 is the only known human antimicrobial peptide in the Cathelicidin familiy. It is widely expressed in both neutrophils and epithelial tissues. Infection and inflammation of epithelial tissues due to a variety of causes have been shown to induce a marked increase in expression.

Usually, the first point of contact between a (mammalian) body and a microorganism takes place at an epithelial surface. Thus, not surprisingly, the epithelium of mammals, e.g. humans, is capable of mounting its own battery of innate immune effector molecules.

In the skin, these effector molecules are (mainly) produced in the uppermost epidermal cell layer.

Some of these molecules, including lysozyme, secretory leukoprotease inhibitor or antileukoprotease, RNase 7 and dermcidin, are constitutively produced by healthy skin keratinocytes or sweat duct cells. Other antimicrobial peptides, such as human β-Defensin 2 and β-Defensin 3 as well as LL-37, are locally induced in skin keratinocytes and after infection or inflammation.

Recently another protein called Psoriasin which has originally been identified in the keratinocytes of the psoriatic epidermis (Madsen et al. (1991) J. Invest. Dermatol. 97(4): 701-12) has been shown to exhibit antimicrobial activity (Gläser et al. (2004) Nat. Immunol. 6, 57-64).

Despite, as briefly outlined, very complex and fascinating defence mechanisms against microbial infections have evolved, microbial infections are widespread.
Tremendous progress has been made in developing antibiotics combating these infections. Unfortunately, use of these drugs can lead to resistance of the microorganism to these drugs as well as to various side effects in the patient.

The object of the present invention is to provide a support of the antimicrobial activity of the innate immune system.

Surprisingly it was found that the application of compositions comprising urea results in an increased expression of genes encoding antimicrobial peptides.

Accordingly, the present invention relates to the use of a composition comprising urea for increasing the expression of at least one gene encoding an antimicrobial peptide.

The term "antimicrobial peptides" according to the present invention relates to peptides of the innate immune systems displaying antimicrobial activity, i.e. these peptides are involved in inactivating microorganisms. The effect of an individual antimicrobial peptide might be restricted to a small number of microorganisms. Alternatively the individual antimicrobial peptide might affect a broader range of microorganisms.

Preferably said term relates to such peptides of mammalian origin, more preferably of human origin.

The term "antimicrobial activity" in its strict sense refers to the effect of the direct interaction of the antimicrobial peptides with the microorganism.

In addition, the antimicrobial peptides, namely defensins and the cathelicidins, trigger other effects with are associated with defending the organism against microorganisms.

As well known in the art, the specific set of effects triggered by an antimicrobial peptide is different for the individual antimicrobial peptides.
For the human LL-37 these effects comprise e.g. antiseptic activity, chemoattraction and promotion of angiogenesis and of wound healing, in particular wound neovascularization and re-epithelization of healing skin (Heilborn et al., J. Invest. Dermatol. 120: 379-89; Koczulla et al., J. Clin. Invest. 111: 1665-72).

Preferably the gene according to the invention is a gene encoding a peptide selected from the group comprising of defensins, cathelicidins and psoriasins.

The term "Defensin" according to the present invention includes α-Defensin, β-Defensins and θ-Defensins, preferably the term refers to β-Defensin of which several have been identified at present (see e.g. Schutte et al, Proc. Natl. Acad. Sci. USA, Vol. 99: 2129-2133).
More preferably, the term refers to the human β-Defensin 1 (also named HBD-1), β-Defensin-2 (also named HBD-2) and β-Defensin-3 (HBD-3).
The Genbank-Accession numbers of said defensins are as follows:
HBD-1: NM_005218; HBD-2: AF040153; HBD-3: P81534

The term "Cathelicidin" according to the present invention refers to all members of the cathelicidin-family in mammals. Preferably the term refers to the human cathelicidin LL-37.
The GenBank-Accession numer of LL-37 is NM_004345

The term "Psoriasin" according to the present invention refers to all members of the cathelicin-family in mammals. Preferably the term refers to the human Humpsor.
The GenBank-Accession numer of Humpsor is M86757

In a preferred embodiment of the present invention, the expression of any of said gene is increased by a factor of at least 2, preferably of at least 3, preferably of at least 5, more preferably of at least 10, most preferably of at least 25.

As known to the person skilled in the art, various methods are available to determine the rate of expression of a gene.
Inventively said factor is determined by real time PCR as outlined in the section "Example".

Preferably, said compositions comprising urea are applied topically onto the skin or are administered by injection or by a depot, or administered orally.

Thus, in a preferred embodiment, the invention relates to the use of a composition comprising defined concentrations of urea for increasing the expression of an antimicrobial peptide in the skin.

The skin of mammals, especially human beings is a complex organ being subdivided into many different layers. Each layer serves a specific purpose and thus expresses specific markers and comprises specific structures.

The outer layers of the skin collectively constitute the epidermis. It is the thinnest arrangement of layers of the skin and has the important function to moisturize and protect. More specifically, the epidermis protects the body from desiccation and the invasion of noxious substances, UV light, virus and bacteria. The epidermis is subdivided (from superficial to deep (bordering the dermis)) in the stratum corneum, stratum lucidum (only present at palms and soles), stratum granulosum, stratum spinosum and stratum germinativum (also known as stratum basale).

The layer underneath the epidermis is known as the dermis and is tightly connected to the epidermis by a basement membrane.

The dermis comprises of connective tissue and contains numerous nerve endings that provide the sense of touch and heat. The dermis is also the layer of the skin, wherein hair follicles, sweat glands, sebaceous glands, apocrine and blood vessels are located. The latter also provides nutrients for the epidermis which is not directly linked to the blood system.

The term "skin" as used according to the present invention preferably relates to the skin of mammals, more preferably of humans.
In one embodiment the term "skin" also includes the mucosa and the nails.
In one embodiment it also includes the epidermis in the nose as well as in the oral cavity, oesophagus and the intestinal system.
Thus, the term "skin" as used herein includes the epidermal surface of e.g. feet, legs, trunk, arms, hands, necks, head, oral cavity, nose, intestinal system, oesophagus, mucosa, genitals, nails and hair.

The inventively used compositions can either be injected directly into the skin and/or in another tissue, preferably into connective tissue, more preferably into a region of connective tissue which is less than 1 cm distant to the skin.

Although the skin is a major organ in defending the organism against microbial infections, antimicrobial peptides are being expressed also in cells of other organs/tissues of the organism.

These cells and/or tissues/organs are, with respect to a-defensins, e.g. neutrophils, NK, cells, T-lymphocytes and Paneth cells of the small intestine.
With respect to the β-defensins these cells and/or tissues/organs include e.g. tongue, skin, cornea, salivary glands, kidney, esophagus, respiratory tract.
With respect to cathelicidin these cells and/or tissues/organs include e.g. neutrophils, epithelial cells and macrophages.

Thus, in another embodiment, the composition used according to the invention is administered by injection and/or by injection of a depot to the organism, preferably into a tissue/organ of the organism physiologically expressing antimicrobial peptides, and/or orally.

Surprisingly it was found, that compositions comprising defined concentrations of urea are particularly effective with respect to the inventive use.

Thus, in a preferred embodiment, the inventively used composition comprises 1 to 30% by weight, based on the total amount of the composition, of urea.

In a more preferred embodiment, the inventively used composition comprises at least 2% by weight, preferably at least 5% by weight, more preferably at least 10% by weight, even more preferably at least 15% by weight, most preferably at least 18% by weight, based on the total amount of the composition, of urea.

In another more preferred embodiment, the inventively used composition comprises at most 25% by weight, preferably at most 22% by weight, more preferably at most 15% by weight, based on the total amount of the compostion, of urea.

Most preferably, the inventively used composition comprises 5% to 20%, preferably 10% to 20% by weight, based on the total amount of the composition, of urea.

The term "composition" as used according to the present invention comprises but is not limited to cosmetic and pharmaceutical compositions.

To achieve an optimal effect of the amount of urea present in the inventively used composition, the other components of said compositions need to be selected and composed accordingly.

The person skilled in the art will be able to specifically adapt the inventively used composition according to the intended application/administration to the organism, preferably to the skin. Furthermore the particular combination of components in the composition will depend on the type of application, the specific purpose being addressed by the composition and the like.

Exemplary formulations of application/administration are outlined further below.

When applied/administered to the skin, the person skilled in the art will also be able to specifically adapt the composition according to the invention with respect to any regions of the skin to which said composition should be administered.

Thus, the person skilled in the art will be able to select and/or avoid auxiliary substances which might be suitable or unsuitable for use in the different regions of the skin, e.g. eyes, face, neck, arms, legs etc.

The selection of components for an inventively used composition has also to take into account that such component does not infer with the effect of urea.
Subject to these provisions and depending on the specific requirements of the application, the inventively used compositions may contain one or more of the following of compounds: gelling agents, oils, waxes, thickening agents, hydrophilic or hydrophobic polymers, moisturizers, emulsifying agents, emollients, fatty acids, organic solvents, stabilizers, sequestering agents, acidifying or basifying agents, surfactants, film formers, biological additives to enhance performance and/or consumer appeal such as amino acids, e.g. isoleucine, proteins, vanilla, aloe extract or bioflavinoids, buffering agents, chelating agents such as ethylenediaminetetraacetic acid (EDTA) or oxalic acid, colorants, dyes, propellants, antifoaming agents, wetting agents, vitamins, emulsion stabilizers, pH adjusters, thickening agents, fragrances, fillers, preservatives, opacifying agents, water and/or alcohols.
An inventively used composition including both urea and isoleucine is preferred. The aforementioned auxiliary agents for the inventively used compositions are used in the usual amounts known by those skilled in the art.
As oils for the inventively used compositions oils from animal or vegetable sources or synthetic are preferably used. As oils can be used at least one oil selected from the group comprising mineral oil, liquid petrolatum, liquid paraffin, volatile and non-volatile silicone oils, isoparaffins, polyalphaolefins, fluorated and perfluorated oils. Examples of further suitable oils - depending on the compounds of the composition - comprise hydrocarbon-based oils of animal origin such as squalene, perhydrosqualene; hydrocarbon-based plant oils such as liquid triglycerides of fatty acids of 4 to 10 carbon atoms, for instance, heptanoic or octanoic acid triglycerides, or oils such as olive oil, macademia nut oil, sunflower oil, sunflower seed oil, com oil, soybean oil, grapeseed oil, castor oil, avocado oil, caprylic/capric acid triglycerides, jojoba oil; linear or branched hydrocarbons of mineral or synthetic origin such as liquid paraffins and derivatives thereof, petroleum jelly; synthetic esters and ethers, in particular esters of fatty alcohols, like; for example, octyldodecyl myristate, glyceryl octanoate, ethylhexyl isononanoate,isopropyl myristate, 2-ethylhexyl palmitate, 2-octyldodecyl stearate, isostearyl isostearate; hydroxylated esters such as isostearyl lactate, octyl hydroxystearate, octyldodecyl hydroxystearate, heptanoates, octanoates and decanoates of fatty alcohols; polyol esters such as propylene glycol dioctanoate, neopentyl glycol diheptanoate, diethylene glycol diisononanoate, and pentaerythritol esters; fatty alcohols containing from 12 to 26 carbon atoms such as octyldodecanol, 2-butyloctanol, 2-hexyldecanol, 2-undecylpentadecanol, oleyl alcohol; partially hydrocarbon-based fluoro oils and/or fluorosilicone oils; silicone oils such as volatile or non-volatile, linear or cyclic polymethylsiloxanes (PDMS) that are liquid or semisolid at room temperature such as cyclomethicones and dimethicones, optionally comprising a phenyl group, for instance phenyl trimethicones, siloxanes, and mixtures thereof.

The oil phase of the inventively used compositions may comprise one or more waxes, gums, or mixtures thereof. The waxes include hydrocarbon-based waxes, fluoro waxes and/or silicone waxes and can be of plant, mineral, animal and/or synthetic origin.
The gums are e.g. high molecular weight PDMSs, cellulose gums or polysaccharides, and the semi-solid materials are generally hydrocarbon-based compounds, such as, but not limited to, lanolins and derivatives thereof, or alternatively PDMSs.

The term "moisturizer" as used according to the present invention means a compound that acts on the barrier function, in order to maintain the moisturization of the stratum corneum, or an occlusive compound. Mention may be made of ceramides, sphingoid-based compounds, lecithins, lycosphingolipids, phospholipids, cholesterol and derivatives thereof, phytosterols (stigmasterol, β-sitosterol or campesterol), essential fatty acids, 1,2-diacylglycerol, 4-chromanone, pentacyclic triterpenes such as ursolic acid, liquid petroleum jelly and lanolin; or a compound that directly increases the water content of the stratum corneum, such as threalose and derivatives thereof, hyaluronic acid and derivatives thereof, glycerol, pentanediol, sodium pidolate, serene, xylitol, sodium lactate, polyglyceryl acrylate, ectoin and derivatives thereof, chitosan, oligosaccharides and polysaccharides, cyclic carbonates, N-lauroylpyrrolidonecarboxylic acid and N-α-benzoyt-L-arginine; or a compound that activates the sebaceous glands, such as DHEA, 7-oxide and/or 17-alkyl derivatives thereof, sapogenins and vitamin D and derivatives thereof.

As stabilizers for the inventively used compositions preferably non-ionic, anionic, cationic and amphiphilic tensides can be used, which are preferably selected from the group comprising polyethylenglycol (PEG) and derivatives thereof, tweens, tritons, spans, polyglycerines, polyalkyl glycerides, alkyl sulfonates, aryl sulfonates, alkyl phosphates, derivatives of alkyl-betaine and phosphatidylglycerole.
Emulsifiers are preferably used in certain formulations of the inventively used compositions in amounts effective to provide uniform blending of ingredients of the composition. Useful emulsifiers include anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium acetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and acetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate. Preferred emulsifiers are glycerol stearate, PEG-40-stearate, sodium carbomer, acrylate/C10-C30-alkyl acrylate crosspolymer.
Surfactants can be used in certain formulations of the inventively used compositions. Suitable surfactants are for example those surfactants generally grouped as cleansing agents, emulsifying agents, foam boosters, hydrotropes, solubilizing agents, suspending agents and non-surfactants, which facilitate the dispersion of solids in liquids. Examples of the surfactants include sorbitan sesquioleate, polysorbate 60, glyceryl stearate, lipophilic glyceryl stearate, sorbitan oleate, sorbitan stearate, DEA-cetyl phosphate, sorbitan stearate/sucrose cocoate, glyceryl stearate/polyethylene glycol- 100 stearate, ceteareth-6 olivate, arachidyl alcohol/behenyl alcohol/arachidyl glucoside, polypropylene glycol- 26-buteth-26/polyethylene glycol-40, and hydrogenated castor oil. Glycerin, sorbitol, propylene glycol, butylene glycol, hexylene glycol, diglycerin, betaine, glycereth-26, or methylgluceth-20 can be used as humectants. Cetyl alcohol, stearyl alcohol, octyl dodecanol, and isostearyl alcohol can be used as higher alcohols.

Xanthan Gum, carbomer, magnesium aluminium silicate, cellulose gum, and/or dextrin palmitate can be used as thickeners. Disodium EDTA and tetrasodium EDTA can be used as chelating agents. Blue No. 1, Red No. 40, and Yellow No. 4 and 5 can be used as colorants.
Suitable film formers which are preferably used in the formulations of the inventively used compositions should keep the composition smooth and even and are preferably, without limitation, at least one substance selected from the group comprising acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (vinyl methylether/maleic acid anhydride copolymer); PVP (polyvinylpyrrolidone); maleic acid anhydride polymer, vinylpyrrolidon/hexadecene copolymer; acrylate copolymer and the like.
The person skilled in the art will be able to adjust the pH-value of the inventively used composition to a suitable pH-value for the particular application.

pH adjusters may also be used in certain formulations of the inventively used compositions. These pH adjusters preferably comprise, but are not limited to ammonium hydroxide, triethanolamine or citric acid.

As further thickening agents used for the inventively used compositions can preferably be used candelilla, carnauba, and microcrystalline waxes, crosslinked acrylic-acid polymers, carbomer, methylhydroxyethylcellulose, hydroxypropylmethylcellulose or hydroxyethylcellulose, and polyethylene thickeners.

Gelling agents, which can be preferably used in the formulations of the inventively used compositions, can be natural or synthetic polymers. Natural polymers are preferably selected from the group comprising Agar-Agar, alginate, pectin, carbomer, carrageenan, casein, dextrine, gelatine, arabic gum, keratine, locust bean gum, xanthan gum and the like. Preferred synthetic polymers, which can be used in the formulations of the inventively used compositions are selected from the group comprising acylic acid polymers, polyacryl amides and alkylene oxide polymers.

Exemplary fillers include but are not limited to talc, silica, zinc stearate, mica, kaolin, nylon (in particular orgasol) powder, polyethylene powder, Teflon, starch, boron nitride, microspheres of copolymers such as Expancel (Nobel Industrie), Polytrap (Dow Coming), and silicone resin microbeads (Tospearl from Toshiba).

The inventively used compositions may also include e.g. a skin penetration enhancer, a skin plumber and/or an optical diffuser.
As skin plumpers a collagen enhancer to the skin can serve. An example of a suitable and preferred skin plumper is palmitoyl oligopeptide. Other skin plumpers are collagen and/or glycosaminoglycan (GAG) enhancing agents. The skin plumper can be preferably present in an amount of from about 0.1 weight % to about 20 weight %, based on the total weight of the composition.
An optical diffuser comprises particles that change the surface optometrics of skin, resulting in a visual blurring and softening of, for example, lines and wrinkles. Examples of such optical diffusers that can be used in the inventively used compositions include, but are not limited to, boron nitride, mica, nylon, polymethylmethacrylate (PMMA), polyurethane powder, sericite, silica, silicone powder, talc, teflon, titanium dioxide, zinc oxide, or any mixtures thereof. An optical diffuser is preferably present in an amount of from about 0.01 weight % to about 20 weight %, based on the total weight of the composition.

The inventively used composition may also include nutritionally desirable minerals.

Other additives include beneficial agents such as those materials that condition the skin (particularly, the upper layers of the skin in the stratum corneum) and keep it soft by retarding the decrease of its water content and/or protect the skin. Such conditioners and moisturizing agents include, by way of example, pyrrolidine carboxylic acid and amino acids; other additives comprise cosmetically and/or cosmeceutically active agents. Such additional cosmetically and/or cosmeceutically active agents include, for example, alpha hydroxyacids, alpha ketoacids, polymeric hydroxyacids, moisturizers, collagen, marine extract.

Other agents may also be added, such as antimicrobial agents, to prevent spoilage upon storage, i.e., to inhibit growth of microbes such as yeasts and molds. Exemplary organic antimicrobrial agents are e.g. 2,4,4'-trichloro-2-hydroxy diphenyl ether (triclosan) and benzoic acid.
The compositions may also contain irritation-mitigating additives to minimize or eliminate the possibility of skin irritation or skin damage resulting from the chemical entity to be administered, or other components of the composition. Exemplary irritation-mitigating additives include, for example: α-tocopherol; monoamine oxidase inhibitors, particularly phenyl alcohols such as 2-phenyl-1-ethanol; glycerin; salicylates; ascorbates; ionophores such as monensin; amphiphilic amines; ammonium chloride; N-acetyloysteine; capsaicin; and chloroquine.

The compositions may also contain insect repellents, self-tanning agents, skin cooling agents, colorants including dyes, lakes and pigments that may be untreated or chemically surface treated to improve wetability or some other property, pearlescent agents, demulcents, agents for stimulating the synthesis of dermal or epidermal macromolecules and/or for preventing their degradation; agents for stimulating fibroblast proliferation and/or for stimulating keratinocyte differentiation; muscle relaxants; tensioning agents; antipollution agents and/or free-radical scavengers, anesthetics, antineoplastics, immune system boosting agents, insect repellents, photostabilizing agents, preservatives, skin protectants, viscosity and/or rheology modifiers.
The pigments which may be added to the inventively used compositions comprise inorganic pigments and organic pigments.

The depigmenting agents that may be incorporated into the composition according to the present invention comprise, for example, the following compounds: kojic acid; ellagic acid; arbutin and derivatives thereof such as those described in U.S. Pat. No. 6,306,376 and U.S. Pat. No. 5,346,693; hydroquinone, without this list being limiting. The composition according to the invention may comprise dermo-decontracting agents, among which mention may be made in particular of alverine and salts thereof, especially alverine citrate, sapogenins such as diosgenin and natural extracts containing it (such as extracts of wild yam), certain secondary and tertiary carbonyl amines, organic or mineral salts of metals, in particular manganese gluconate, adenosine, and also the hexapeptide argireline R sold by the company Lipotec. Mention may also be made of certain fragrancing compositions with a dermo-decontracting effect.

Preferably the inventively used composition comprises optionally at least one compound selected from the group comprising UV-filters, vitamins, coenzymes, retinoids, phytokines and antioxidative agents.

Suitable vitamins are for example vitamin A, vitamins of the vitamin B group, e.g. vitamin B1 and B5, vitamin C, vitamins of the vitamin D group such as for example vitamin D and vitamin D3, and vitamin E. As it will be obvious to the person skilled in the art, the inventively used compositions may (also) comprise suitable derivatives of said vitamins such as the vitamin D-derivative 1-fluoro-25-hydroxy-16-ene-23-yne-hexa:fluoro-cholecalciferol.

An antioxidant functions, among other things, to scavenge free radicals from skin to protect the skin from environmental aggressors. Examples of antioxidants that may be used in the present compositions include compounds having phenolic hydroxy functions, such as ascorbic acid and its derivatives/esters e.g. sodium-L-ascorbate, calcium-L-ascorbate, ascorbyl palmitate; beta-carotene; catechins; curcumin; ferulic acid derivatives (e.g. ethyl ferulate, sodium ferulate); gallic acid derivatives (e.g. propyl gallate); lycopene; reductic acid; rosmarinic acid; tannic acid; tetrahydrocurcumin; tocopherol and its derivatives e.g. α-tocopherol, γ-tocopherol, δ-tocopherol; uric acid; or any mixtures thereof. Also propylgallate, octylgallate, dodecylgallate, sodium-isoascorbate, citric acid, sodium citrate, potassium citrate and tin-II-chloride. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur.

Retinoids which can be used additionally include, without limitation, retinoic acid (e.g., all-trans or 13-cis) and derivatives thereof, retinol (Vitamin A) and esters thereof, such as retinol palmitate, retinol acetate and retinol propionate, and salts thereof.

In addition, it is also possible to include known agents which stimulate proliferation of keratinocytes and can be formulated with the compositions used according to the invention.

Furthermore, the inventively used composition may include known agents which facilitate the expression of collagen.

Furthermore, the compositions used according to the present invention may include sunscreens. Such sunscreens could be particulate materials, among them are zinc oxide, titanium oxide, clays and ferric chloride. Because such material might be visible and occlusive, many people consider the corresponding opaque formulations cosmetically unacceptable. Other sunscreens rely on chemicals such as p-aminobenzoic acid (PABA), oxybenzone, dioxybenzone, ethylhexyl-methoxy cinnamate, octocrylene, octyl methoxycinnamate, and butylmethoxy-dibenzoylmethane that are transparent or translucent on the skin. While these materials may be more acceptable cosmetically, they are still relatively short-lived and susceptible to being removed by washing or perspiration.

Therefore improved UV-filters have been developed which can be used as auxiliary compounds like the UV-filters preventing/treating signs of aging of the skin disclosed e.g. in: W007025599A1 "Sunscreen composition comprising a dibenzoylmethane, an aminohydroxybenzophenone, a triazine and a triazole as UV filters"; W007002047A1 "A product release system to atomize cosmetic hair or skin compositions containing UV filters"; WO06032741A1 "Silane merocyanine sulphone derivatives; photoprotecting compositions containing same; use thereof as UV filter"; WO05094772A3 "Use of benzophenone UV filters for preventing tanning"; W005094772A2 "Use of UV filters for preventing tanning"; WO04082580A3" photostabilized topical formulations of ketoprofen containing two UV filters"; W004022015A1 "Use of diketopiperazine derivatives as photostable UV-filters in cosmetic and pharmaceutical preparations"; WO03039447A3 "Composition containing an amino acid n-acylated ester and a polyamide-structured UV fitter"; WO03039447A2 "Composition containing an amino acid n-acylated ester and a polyamide-structured UV filtet"; WO0239972A1 "Insoluble organic UV filters and cosmetic use thereof"; WO0149686A1 "s-triazine derivatives and their use as UV filters"; WO0126618A3 "Composition, especially a cosmetic composition, containing a steroid and a liposoluble UV fitter"; WO0126618A2 "composition, especially a cosmetic composition, containing a steroid and a liposoluble UV filter"; WO9825922A1 "Insoluble s-triazine derivatives and their use as UV filters".
The disclosed UV-filters in these publications are also incorporated by reference herein as possible components in the urea-containing compositions.

Preferably as one component in the urea-containing compositions the UV-filter is a benzotriazolylphenole or a derivative thereof, preferably a silylated derivative, capable of filtering UV-light and/or a pyrroltriazine or a derivative thereof capable of filtering UV-light and/or a heptaazaphenalene or a derivative thereof capable of filtering UV-light.

Such UV-filters are disclosed in the documents WO2008/131921 "Silylated, substituted benzotriazolylphenol compounds" and in WO2006/128791 "New derivatives of pyrrolyltriazine together with methods for obtaining them and their use as protecting agents against UV radiation" and in WO2007/006807 "New derivatives of heptaazaphenalene, methods for obtaining them, and theirs use as protecting agents against UV radiation" respectively which are incorporated by reference herein.

As it will be obvious to the person skilled in the art, the composition may contain many more compounds suitable for the specific requirement. Some further categories of compounds are disclosed herein after.

Depending on the specific constitution of any of the inventively used compositions, the person skilled in the art will be able to select the appropriate steps for preparing said composition.

The particular combination of components in the composition is determined largely by chemical compatibility. The person skilled in the art will be able to easily determine which of the compounds listed above and which alternative compounds known to the person skilled in the art might be used/combined for a specific composition according to the invention. In particular, all these components listed herein as well as any combination thereof have to fulfill the requirement not to interfere with the effect of urea.

The person skilled in the art will be able to specifically adapt the inventively used composition for use within the oral cavity. These compositions are referred to as "oral compositions" herein.

The term "oral cavity" as used herein is to be understood broadly and includes also e.g. the lips, the pharynx, the jaw and other tissue within or directly surrounding the mouth.

In a preferred embodiment said oral-composition includes an orally acceptable carrier.

The term "orally acceptable carrier" as used herein includes safe and effective materials for use in the compositions of the present invention. Such materials are conventional additives in oral care compositions including but not limited to fluoride ion sources, anti -calculus or anti-tartar agents, buffers, abrasives such as silica, bleaching agents such as peroxide sources, alkali metal bicarbonate salts, thickening materials, humectants, water, surfactants, titanium dioxide, flavor system, sweetening agents, xylitol, coloring agents, and mixtures thereof.

The carriers or excipients of the present invention can include the usual and conventional components of dentifrices, non-abrasive gels, subgingival gels, mouthwashes or rinses, mouth sprays, chewing gums, lozenges and breath mints as more fully described hereinafter.

The choice of a carrier to be used is basically determined by the way the composition is to be introduced into the oral cavity. Carrier materials for toothpaste, tooth gel or the like include abrasive materials, sudsing agents, binders, humectants, flavoring and sweetening agents, etc. as disclosed in e.g., U.S. Pat. No. 3,988,433, to Benedict. Carrier materials for biphasic dentifrice formulations are disclosed in U.S. Pat. Nos. 5,213,790, issued May 23, 1993, 5,145,666, issued September 8, 1992, and 5,281,410 issued January 25, 1994 all to Lukacovic et al. and in U. S. Patents 4,849,213 and 4,528,180 to Schaeffer. Mouthwash, rinse or mouth spray carrier materials typically include water, flavoring and sweetening agents, etc., as disclosed in, e.g., U.S. Pat. No. 3,988,433 to Benedict. Lozenge carrier materials typically include a candy base; chewing gum carrier materials include a gum base, flavoring and sweetening agents, as in, e.g., U.S. Pat. No. 4,083,955, to Grabenstetter et al. Sachet carrier materials typically include a sachet bag, flavoring and sweetening agents. For subgingival gels used for delivery of actives into the periodontal pockets or around the periodontal pockets, a "subgingival gel carrier" is chosen as disclosed in, e.g. U.S. Pat. Nos. 5,198,220 and 5,242,910, issued March 30, 1993 and Sept. 7, 1993, respectively both to Damani. Carriers suitable for the preparation of compositions of the present invention are well known in the art. Their selection will depend on secondary considerations like taste, cost, and shelf stability, etc.

The oral compositions of the present invention will optionally include a soluble fluoride source capable of providing bioavailable and efficacious fluoride ions.

Dental abrasives useful in the oral compositions include many different materials. The material selected must be one which is compatible within the composition of interest and does not excessively abrade dentin. Suitable abrasives include, for example, silicas including gels and precipitates, insoluble sodium polymetaphosphate, hydrated alumina, calcium carbonate, dicalcium orthophosphate dihydrate, calcium pyrophosphate, tricalcium phosphate, calcium polymetaphosphate, and resinous abrasive materials such as particulate condensation products of urea and formaldehyde.

The present compositions may contain a buffering agent.

The inventively used compositions, preferably the oral compositions, may also include flavoring agents, sweetening agents, titanium dioxide, coloring agents, all of which are preferably suitable for oral use.

As outlined, the compositions comprising urea are preferably topically applied onto the skin or administered by injection.

For the preferred topical application, the inventively used composition may be formulated in liquid or in semi-solid form, preferably as liquid, fluid, foam, cream, gel, paste, balsam, spray, ointment, lotion, conditioner, tonic, milk, mousse, emulsion, serum, oil, stick, shampoo, jelly, suspension, dispersion, lacquer, paint, elixir, drop, aerosol, day creams or lotions, night creams or lotions, salves, sunscreen creams, fluid lotions, oils, water-in-oil, oil-in-water, water-oil-water triple emulsions, masks, body milks, makeup, artificial tanning compositions, depilatories, emulsifiers, patches, or a solid which is poured or cast as a stick or a dish, microemulsions, aerosol, powder, soap, surfactant-containing cleansing powder foundation, emulsion foundation, wax foundation, softening lotion, nutrient lotion, nutrient cream, massage cream, bath compositions, aftershave gels or lotions, cleansing cream, cleansing foam, cleansing water or packs, composition for countering insect stings or bits, pain-relieving compositions or compositions for treating certain skin diseases or skin disorders. It is also contemplated that topical compositions of the present invention can be incorporated into delivery systems such as liposomes, nanoparticles, topical patches, tapes and sprays. It may also be formulated in a makeup product for facial skin, the body or the lips, such as a foundation, a tinted cream, a makeup rouge, an eyeshadow, a loose or compact powder, a concealer stick, a cover stick, a lipstick or a lipcare product.

As far as the invention relates to a topical application, it is referred to International Cosmetic Ingredient Dictionary and Handbook (ICID), 10th Edition (2004), published by the Cosmetic, Toiletry, and Fragrance Association (CTFA), at pp. 2177-2299, which includes information about suitable ingredients and their use and which is herein incorporated by reference in its entirety.

For administration by injection, preferably into the skin, the composition can be provided in the form of aqueous or oily lotions or in the form of serums.

Preferably, the pH-value of the inventively used composition will be adjusted using a suitable puffering agent. Preferably the pH-value of the inventively used composition will be in the range of 3,5 - 9,0 for topical application.

For injection, the preferred pH-value of the inventively used composition will be in the range of 3,0 - 9.0.

The oral composition of the present invention may be in various forms including toothpaste, tooth gel, dentifrice, tooth powder, topical oral gel, mouthrinse, mouthwash, denture product, mouthspray, mousse, foam, lozenge, oral tablet, and chewing gum, non-abrasive gels; dental solutions and irrigation fluids; and dental implements, The dental implement can be impregnated fibers including dental floss or tape, chips, strips, films and polymer fibers.

The present compositions may also be delivered to tissues and/or spaces within the oral cavity using electromechanical devices such as metering devices, targeted application devices and cleaning or integrated oral hygiene systems.

For treating oral tissue wounds and aiding tissue regeneration, fluid subgingival gel compositions that can be inserted via syringe and either a needle or catheter directly into the areas needing treatment, such as the periodontal cavities, may be used.

The composition used according to the invention might be applied to the complete skin or at least significant parts thereof.
The application might also be limited to specific regions of the body which are particularly vulnerable for microbial infections, which are infected or which are in the proximity of regions which are infected.

For a successful treatment of the skin the compositions comprising urea is applied onto the skin at least once a day for at least 2 weeks topically and/or administered by injection and optionally longer until the expression of any of said genes is increased by a factor of at least 2, preferably at least 3, more preferably at least 5, even more preferably of at least 10, most preferably of at least 25.

A successful treatment of the skin can also be achieved by injections, preferably by, optionally repeated, injections of a depot, or administered orally.

In total the period of application and/or administration can continue for several weeks or even several months or even several years either continuously or in repeated phases of application.

In general the method of delivery of the active agent may vary, but necessarily involves application of the inventively used composition to the area of the skin were the desired effect is intended.

As indicated before, the application and/or administration of the inventively used compositions results in an increase in the expression of genes encoding antimicrobial peptides.

Thus the present invention provides a support of the antimicrobial activity of the innate immune system.

Thus, one further embodiment of the present invention relates to the use of a composition according to the present invention for preventing and/or treating a disease in which microorganisms have an influence.

The term "microorganism" according to the present invention is to be understood broadly and comprises bacteria (both gram-negative and gram-positive bacteria), archaebacteria, viruses, in particularly enveloped viruses, fungi and yeast.
In particularly, the term refers to species, variants and the like of the aforementioned microorganisms which are pathogenic to mammalians. The term even includes transformed or cancerous cells.

Thus, in a preferred embodiment the present invention relates to said use in which the microorganism is selected from the group comprising gram-negative bacteria, gram-positive bacteria, archaebacteria, viruses, fungi, yeast, parasites, nematodes, transformed and cancerous cells.

Exemplary bacterial genuses including bacteria infecting humans are: Bordetella, Borrelia, Brucella, Campylobacter, Chamydia, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema, Vibrio and Yersinia.

Several microorganisms may be found in the oral cavity. These include e.g. Porphyromonas gingivalis, B. forsythus, A. actinomycetemcomitans, T. denticola, T. socranskii, F. nucleatum, and P. intermedia and against other oral cavity strains such as L. acidophilus, L. casei, A. viscosus, S. sobrinus, Streptococcus sanguis, S. viridans, and S. mutans.

Further exemplary microorganisms are described e.g. in "Medical Microbiology", 24th edition by Geo. Brooks, Karen C. Carroll, Janet Butel, Stephen Morse or in Manual of Clinical Microbiology, 7th edition, Patrick R., Ph.D. Murray, Ellen Jo Baron, James H. Jorgensen, Marie Louise Landry, Michael A. Pfaller.

The term "organism" as used herein refers to an organism which can be infected by an microorganism. Preferably, the term refers to a mammal, more preferably a human being.

The term "disease in which microorganisms have an influence" according to the present invention is to be understood broadly.
It includes all symptoms, impairments of any physiological process and the like (either temporarily for 1 to 30 days, 1 to 12 months or longer, or permanently) which presence or degree correlates with a contact of the organism to a microorganism. Said contact may have taken place at any time, preferably at the point in time at which said symptoms, impairments and the like take place.

In one embodiment, the occurrence of said symptoms is due to any additional "burden" to the organisms resulting from being contacted by a microorganism.
Said "burden" includes but is not limited to an activation of the immune systems which might facilitate allergic reactions, have an impact on the physiological colonization of the organism by microorganisms (e.g. the physiological gut flora) or reduce the ability of the organism to combat other pathological processes and the like.

In a preferred embodiment, the term "disease in which microorganisms have an influence" relates to diseases which are caused by microorganisms.

The term "caused by microorganisms" as used according to the invention refers to a disease for which an infection by a microorganism is a necessary requirement.

In a preferred embodiment, the disease is caused by a microorganism selected from the group comprising gram-negative bacteria, gram-positive bacteria, archaebacteria, viruses, fungi, yeast, parasites, nematodes, transformed and cancerous cells.

The term "preventing" according to the present invention refers to reduction, preferable the avoidance, of a risk of obtaining a disease in which microorganisms have an influence, preferable a disease which is caused by microorganisms. The term also refers to reducing the risk of a microbial infection or reducing the severity of a putative microbial infection.

The term "treating" according to the present invention comprises preventing, inhibiting, curing, and alleviating the disease or symptoms thereof.

Exemplary diseases which are caused by microorganisms are spirochaetal infection, malaria, amoebic nondysenteric colitis, acanthamoebiasis, acariasis, acarine dermatitis, actinomycetoma, actinomycosis, actinomycotic infections, acute amoebic dysentery, acute amoebic dysentery without mention of abscess, acute and fulminating melioidosis, acute meningococcaemia, acute poliomyelitis, adenovirus infections, adiaspiromycosis, african trypanosomiasis, AIDS, ainhum, allescheriasis, amebiasis, amoebic abscesses, amoebic nondysenteric colitis, amoebic skin ulceration, amoeboma of intestine, ancylostomiasis and necatoriasis, angiostrongyliasis due to parastrongylus cantonensis, anisakiasis, anthrax infections, arbovirus infections, argentine hemorrhagic fever (Junin virus), arthropod-borne hemorrhagic fever, ascariasis, aseptic meningitis, aspergillosis, babesiosis, Bacillary dysentery, Bacillus fragilis (B. fragilis) infections, bacterial meningitis, balantidiasis, Barmah Forest, bartonellosis, beard ringworm infection, behcet's syndrome, bejel, black piedra, blastomycosis, bolivian hemorrhagic fever, bomholm disease, botulism, boutonneuse fever, bovine stomatitis, brazilian purpuric fever, brucellosis, bubonic plague, buruli ulcer, california encephalitis virus infection, campylobacter enteritis, campylobacter infection, campylobacteriosis, campylobacteriosis, cancrum oris, candidal stomatitis, candidiasis, capillariasis, cat scratch disease, cercarial dermatitis, Chagas disease, chancroid, chickenpox, chiggers, chikungunya virus disease, chlamydia infections, chlamydial lymphogranuloma (venereum), cholera, chromomycosis and phaeomycotic abscess, chronic intestinal amoebiasis, chronic meningococcaemia, clonorchiasis, clostridium perfringens (C. perfringens) infections, CMV disease, coccidioidomycosis, Colorado tick fever, common cold, condyloma acuminata, coronavirus infections, cowpox and paravaccinia, coxsackievirus infections, Crimean-Congo haemorrhagic fever, cryptococcosis, cryptosporidiosis, cutaneous amoebiasis, cutaneous and mucocutaneous bartonellosis, cutaneous mycobacterial infection, cyclospora, cysticercosis, cytomegalovirus infection, dengue fever, dengue haemorrhagic fever, dermatomycosis, dermatophytic onychia, dermatophytosis, dermatophytosis of nail, diarrhoea and gastroenteritis of presumed infectious origin, dicrocoeliasis, diphtheria, diphyllobothriasis, diseases due to other mycobacteria, donovanosis, dracunculiasis, dwarf tapeworm, dysentery, eastern equine encephalitis, ebola, echinococcosis, echovirus infections, eczema herpeticum, ehrlichiosis, encephalities, endemic syphilis, enteritis due to Yersinia enterocolitica, enterobiasis, enterocolitis due to Clostridium difficile, enteropathogenic Escherichia coli infection, enteroviral vesicular pharyngitis, enteroviral vesicular stomatitis with exanthem, enterovirus infections, epidemic myalgia, epidemic polyarthritis and exanthema, epidemic Typhus, erysipelas, erysipeloid, erysipelothrix infection, erythema chronicum migrans due to Borrelia burgdorferi, erythema infectiosum (fifth disease), eumycetoma, exanthema subitum (sixth disease), external hirudiniasis, fascioliasis, fasciolopsiasis, fifth disease, filarial infection and dracontiasis, filariasis, food poisoning, foodborne Clostridium perfringens (Clostridium welchii) intoxication, foodborne staphylococcal intoxication, foot-and-mouth disease, fusospirochaetal gangrene, gas gangrene, genital herpes, geotrichosis, geotrichum stomatitis, giardiasis, glanders, gnathostomiasi, gonococcal infections, gonorrhea, granuloma inguinale, haemolytic uraemic syndrome, haemophilus influenzae, hand, foot and mouth disease, Hansen's disease (Leprosy), hantavirus infection, helminthiases, hemolytic uremic syndrome, hepatitis A, hepatitis B, hepatitis C, hepatitis D, hepatitis E, herpangina, herpes simplex, herpes zoster, herpetic gingivostomatitis, histoplasmosis, hookworm diseases, HPV, hymenolepiasis, ichthyoparasitism due to Vandellia cirrhosa, impetigo, infection due to Mycobacterium, infection due to Pseudallescheria boydi, infectious mononucleosis, influenza, internal hirudiniasis, intestinal infection due to other organism, intestinal trichomoniasis, isasporiasis, Japanese encephalitis, kala-azar, keloidal blastomycosis, kerion, Kew garden fever, Klebsiella pneumoniae (K. pneumoniae) infections, Kunjin virus, Kyasanur forest disease, lassa fever, leech infestation, legionellosis, Legionnaires' disease, leishmaniasis, lentivirus infections, leprosy, leptospirosis, linguatulosis, listeriosis, lobomycosis, Lobo's disease, loiasis, louse-borne (epidemic) typhus, lyme disease, lymphogranuloma venereum, lyssavirus, Machupo haemorrhagic fever, madura foot, maduromycosis, mansonelliasis, Marburg hemorrhagic fever, Marburg virus disease, measles, melioidosis, meningitis, meningococcaemia, meningococcal disease, metagonimiasis, microsporidiosis, molluscum contagiosum, moniliasis, monkeypox, mononucleosis, mosquito-borne viral encephalitis, mucomysosis, mucopuruient cervicitis, mumps, murray Valley encephalitis virus, mycetoma, mycobacteriosis, mycoplasma infection, myiasis, naegleriasis, necatoriasis, necrotizing ulcerative stomatitis, njovera, nocardiosis, noma, nongonococcal urethritis, norovirus infection, obstetrical tetanus, Omsk haemorrhagic fever, onchocerciasis, oncovirus infections, onychomycosis, ophthalmia neonatorum, o'nyong-nyong fever, opisthorchiasis, opportunistic mycoses, oral thrush, ornithosis, oropouche fever, other helminthiases, other arthropod-bome diseases, other arthropod-bome viral diseases, other atypical virus infections of central nervous system, other cestode infection, other diseases caused by chlamydiae, other diseases due to viruses and Chlamydiae, other forms of bartonellosis, other hookworm diseases, other infestation, other melioidosis, other mosquito-borne viral fevers, other mycoses, other parapoxvirus infections, other poxvirus infections, other protozoal diseases, other protozoal intestinal disease, other rickettsioses, other salmonella infections, other schistosomiases, other septicaemia, other sexually transmitted chlamydial diseases, other specified mosquito-borne viral fevers, other specified protozoal diseases, other spirochaetal infection, other superficial mycoses, other trematode infections, other Vincent's infections, other viral diseases, other viral haemorrhagic fevers, papillomavirus infections, pappataci fever, paracoccidioidomycosis, paragonimiasis, paratyphoid fever, parvovirus infections, pasteurellosis, pediculosis and phthiriasis, pelvic inflammatory disease, penicillosis, pertussis, phlebotomus fever, pinta, pinworm infection, piroplasmosis, piry virus disease, pityriasis versicolor, plague, pneumococcal disease, pneumococcal pneumonia, pneumococcal septicemia, pneumocystosis, poliomyelitis, porocephaliasis, poxvirus infections, primary amoebic meningoencephalitis, progressive multifocal leukencephalopathy, proteus (mirabilis)(morganii) infections, protozoal intestinal disease, pseudomonas (aeruginosa)(mallei)(pseudomallei) infections, psittacosis, psorospermiasis, pulmonary mycobacterial infection, Q fever , Quintan fever, Rabies, Rat-bite fever, Relapsing fever, Reovirus infections, Respiratory syncytial virus, Retrovirus infections, Rheumatic fever, Rhinosporidiosis, Rhinovirus, Rickettsiosis, Rift Valley fever, Ringworm of nails, Rocky mountain spotted fever, Roseola infantum, Ross River fever, Rotaviral enteritis, Rubella, salmonella gastroenteritis, salmonellosis, sandfly fever, sarcocystosis, sarcoidosis, sarcosporidiosis, SARS, scabies, scalp ringworm, scarabiasis, scarlet fever, schistosomiasis, scrofuloderma, sealpox, septicaemia, shigellosis, smallpox (Variola), sparganosis, spirillosis, sporotrichosis, spotted fever, St. Louis encephalitis, stomatitis gangrenosa, strep throat, streptobacillosis, streptococcus and staphylococcus infections, strongyloidiasis, sweating fever, sycosis, syngamiasis, syphilis, taeniasis, tanapox, tetanus, tick-bome rickettsioses, tick-bome viral encephalitis, tinea, toxic shock syndrome, toxocariasis, toxoplasmosis, trachoma, trench fever, trench mouth, trichinellosis, trichinosis, trichomoniasis, trichostrongyliasis, trichuriasis, trombiculosis, trypanosomiasis, tuberculosis, tularemia, tungiasis (sandflea infestation), typhoid and paratyphoid fevers, typhoid fever , typhus, vaccinia , varicella (chickenpox), venezuelan equine fever, verruca, vesicular rickettsiosis, vesicular stomatitis virus disease (Indiana fever), vincent's angina, viral carditis, viral conjunctivitis, viral encephalitis, viral gastroenteritis, viral haemorrhagic fever, including Lassa fever and Marburg virus, viral hepatitis, viral meningitis, viral pneumonia, viral warts, visceral larva migrans, Waterhouse-Friderichsen syndrome, West Nile disease, Western equine encephalitis, White piedra, Whitmore's disease, whooping cough, wolhynian fever, Yaba monkey tumor virus, yatapoxvirus infections, yaws, yellow fever, zygomycosis.

Infections of the oral cavity include e.g. caries (e.g. caries of dentine, caries of cementum, arrested dental carries), acute, gingivitis, chronic gingivitis, gingivostomatitis, Vincent's infection, periodontosis, acute periodontitis, chronic periodontitis, Rigg's disease, pulpitis stomatitis and glossitis.
Furthermore said infections include infections of the salivary glands such as parotitis and sialadenitis.
Inflammatory conditions of the jaws are e.g. osteitis, osteomyelitis, osteoradionecrosis, condensing osteitis, gnathitis and periostitis.
Infections of the lip are e.g. various forms of cheilitis.
Infections of the throat include pharyngitis.

In terms of site of the infection, said infection can affect different tissues of the oral cavity.
The term "tissue of the oral cavity" includes e.g. the enamel of the tooth, the dentin of the tooth, the cementum of the tooth, the pulp of the tooth, the root of the tooth, the gingiva, the alveolar bone, the jaw bone, the tongue, the salivary gland, the lips, the pharynx, the periodontium, the various mucosa of the oral cavity.

Oral and dental infections are more fully disclosed in Finegold, Anaerobic Bacteria in Human Diseases, chapter 4, pp 78-104, and chapter 6, pp 115-154 (Academic Press, Inc., NY, 1977.

All diseases described therein and which are caused by an infection are to be understood as "diseases caused by microorganism" according to the present invention.

Further diseases caused by microorganisms are described e.g. in the following textbooks:
Infectious Diseases (Infectious Diseases (Gorbach )) by Sherwood L Gorbach, John G Bartlett, and Neil R Blacklow (2003); and Principles and Practice of Infectious Diseases by Gerald Mandell, John Bennett, and Raphael Dolin (2004).
All diseases described therein and which are caused by an infection are to be understood as "diseases caused by microorganism" according to the present invention.

Thus, these textbooks are herein incorporated by reference in its entirety.

As described herein, individual antimicrobial peptides exert specific additional effects other than the direct interaction with a microorganism. As described herein, LL-37 for example is involved in antiseptic activity, chemoattraction and promotion of angiogenesis and of wound healing, in particular wound neovascularization and re-epithelization of healing skin (Heilbom et al., J. Invest. Dermatol. 120: 379-89; Koczulla et al., J. Clin. Invest. 111: 1665-72).

Thus, the invention also relates to the use a composition according to the invention for preventing and/or treating a disease and/or damage wherein said disease and/or damage is a disease and/or damage which severity can be reduced by the expression of antimicrobial peptides.

Recently, it has been shown that an imbalance of defensins in the skin may contribute to acne (Philpott (2003) Mol. Immunol. 40: 457-62).
Thus, in a preferred embodiment, the compositions according to the invention may be used for preventing and/or treating of acne.
Furthermore, it has recently been shown that a reduced level of α-defensins contributes to the etiology of Crohn's disease (Wehkamp et al. (2005) Proc. Natl. Acad. Sci. USA 102:18129-34).
Thus, in a preferred embodiment, the compositions according to the invention may be used for preventing and/or treating of Crohn's disease.

The antimicrobial peptides are particularly highly expressed in the epidermis. The epidermis also forms the lining of the surfaces of the body both externally and internally (such as the intestinal wall or the surfaces of the nose and the oral cavity).

In a preferred embodiment, the disease caused by microorganisms is a disease affecting the epidermis.

In a preferred embodiment, the disease according to the invention is a skin disease. Such skin diseases include e.g. atopical dermatitis, neurodermitis, rosacea.

Thus, a preferred embodiment of the present invention relates to the use of a composition according to the present invention for preventing and/or treating a skin disease.

For preventing any of the aforementioned diseases, preferably skin diseases, the inventively used composition may be added to normal skin.

The term "normal tissue" and "normal skin" respectively as used according to the present invention refers to healthy tissue/skin of a mammal, preferably a human being, of the respective age taking into account the sex, ethnic background as well as the part of the body. According to the present invention such normal tissue/skin is not infected by microorganism which causes a disease according to the present invention and/or which presence correlates with a disease according to the present invention.

Very often a microbial infection poses a particular problem for a tissue/organ which is already damaged. In particular, microbial infections pose a particular problem for skin which is already damaged.

The term "damages" as used according to the present invention refers to any damage of a tissue/organ either caused by intrinsic and/or extrinsic factors including e.g. damages caused by mechanical factors, environmental factors such as toxins, damages caused by genetic factors and damages associated with aging. Particularly with respect to the skin, such damages also include damages caused by UV-light. Damages due to mechanical factors include e.g. cuts and abrasions. The term also includes burns.

Furthermore the term "damage" relates to any damages and/or impairments which are caused and/or correlates with any disease of the organisms.

Thus the inventively used composition can be applied both to normal tissue, preferably skin, as well as to already damaged tissue, preferably skin, of a mammal, preferable of a human being.

Various damages may specifically occur in the oral cavity. Due to either direct or indirect effects said damages may increase the risk of the occurrence of a disease caused by a microorganism within the oral cavity and possibly extending beyond the oral cavity.
Said damages are referred to herein as "damages of the oral cavity" and include e.g. anomalies in tooth development, anomalies in tooth position, hypodontia, hyperdontia, malocclusion, abrasions and erosions of teeth, loss of teeth, cysts, tumors, diseases of salivary glands such as sialoadenitis, fistuls, disturbances of salivary secretion, diseases of the tongue, diseases of the jaw, concrescence, abcesses, necrosis.

The term "damages of the oral cavity" also includes the presence of bridges, coronas, implants, inlays, onlays and the like.
The term also includes other damages that may develop following dental procedures such as osseous surgery, tooth extraction, periodontal flap surgery, dental implantation, and scaling and root planing.

Thus, in a preferred embodiment the present invention relates to the use of a composition according to the present invention for the prevention and/or treating a disease caused by microorganism in humans having damages of the oral cavity.

A damage to the skin which serves very often as an entry point for microorganisms causing infections are wounds.

Thus, a further embodiment relates to the inventive use of the composition according to the present invention on tissue, preferably skin, exhibiting wounds.

It could happen that large areas of the tissue/skin are affected in case of scalps and burns. Such large scale wounds pose a particular risk with respect to microbial infections.

Accordingly a further embodiment of the present invention is the treatment of wounds caused by burns.

Bedsores, more properly known as pressure ulcers or decubitus ulcers, are lesions caused by many factors such as: unrelieved pressure; friction; humidity; shearing forces; temperature; age and medication; to any part of the body, especially portions over bony or cartilaginous areas such as sacrum, elbows, knees, ankles etc. Although completely treatable if found early, without medical attention, bedsores can become life-threatening, and indeed fatal. In patients suffering from decubitus ulcers the skin is damaged being painful and posing a risk for infections and other diseases. One of the risk factors for developing decubitus ulcers is a damaged/weak skin.

Therefore a further embodiment is the use of the compositions according to the present invention on skin exhibiting wounds like decubitus ulcer.

It is well known that patients suffering from diabetis have problems with wound healing also because the skin becomes dry and fissured which often leads to wounds. Thus, patients suffering from diabetis are particularly at risk with respect to microbial infections.

A further object of the present invention is the use the compositions according to the present invention to treat wounds of human beings suffering from diabetis.

The composition according to the present invention may also be cosmetically beneficial.

Thus, one further embodiment of the present invention relates to the use of a composition according to the invention adjusted as a cosmetic composition.

For the inventively used composition marketed in Europe as a cosmetic composition the respective components shall be used in amounts which are in compliance with the Council Directives 76/768/EEC and Commission Directive 95/17/EC on the approximation of the laws of the Member States relating to cosmetic products.

In another preferred embodiment the composition according to the present invention is a pharmaceutical composition.

As it has been surprisingly shown that the application of compositions comprising urea results in an increase of the expression of genes encoding an antimicrobial peptide, one embodiment of the present invention relates to a pharmaceutical composition only comprising urea as a pharmaceutically active agent.

In another preferred embodiment, said pharmaceutical composition comprises a least one further pharmaceutically active agent.

Such (further) pharmaceutically active agents are well known in the field.

They include but are not limited to antimicrobial agents other than antimicrobial peptides; antibacterial agents other than antimicrobial peptides; antipruritic and antixerotic agents; antiinflammatory agents, e.g. acetylsalicylic acid and glycyrrhetinic acid.; anti-seborrhoeic agents; vasodilators; inhibitors of melanogenesis; anti-allergenics; antiseptics; nitric oxide synthase inhibitors; agents for stimulating the synthesis of dermal or epidermal macromolecules and/or for preventing their degradation; agents for stimulating fibroblast proliferation and/or for stimulating keratinocyte differentiation; muscle relaxants; tensioning agents; antineoplastics; antivirals; hypopigmenting agents; immune system boosting agents; local anesthetics and analgesics; corticosteroids; and antimetabolites and are at least one active agent selected from the group comprising acyclovir, amphotericins, chlorhexidine, clotrimazole, ketoconazole, econazole, miconazole, metronidazole, minocycline, nystatin, neomycin, kanamycin, phenytoin, pare-amino benzoic acid esters, octyl methoxycinnamate, octyl salicylate, oxybenzone, dioxybenzone, tocopherol, tocopheryl acetate, selenium sulfide, zinc pyrithione, diphenhydramine, pramoxine, lidocaine, procaine, erythromycin, tekacyciine' clindamycin, crotamiton, hydroquinone and its monomethyl and benzyl ethers, naproxen, ibuprofen, cromolyn, retinol, retinyl palmitate, retinyl acetate, coal tar, griseofulvin, estradiol, hydrocortisone, hydrocortisone 21-acetate, hydrocortisone 17- valerate, hydrocortisone 17-butyrate, progesterone, betamethasone valerate, betamethasone dipropionate, triamcinolone - 20 acetonide, fluocinonide, clobetasol propionate, minoxidil, dipyridamole, diphenylhydantoin, benzoyl peroxide, and 5-fluorouracil.

A pharmacological acceptable carrier may also be incorporated in the inventively used composition and may be any carrier conventionally used in the art.

Additional examples of pharmaceutically active agents that can be used as components in the inventively used compositions can be found e.g. in "Rote Liste - Arzneimiffelverzeichnis für Deutschland" (Edition 2008, published by Rote Liste Service GmbH, Frankfurt/Main, Germany) which is incorporated by reference herein.
Additional examples of pharmaceutically active agents for use in dermatology can be found in e.g. "Hauptgruppe 32 'Dermatika'" of the Rote Liste (Edition 2008) which are incorporated by reference herein.
As discussed before, examples of antibiotics, anti-infectives and pharmaceutically active agents against dermatoses are well known in the field.
Examples of such pharmaceutically active agents can also be found e.g. in the Rote Liste which is, as outlined before, incorporated by reference herein.
Specifically, examples of antibiotics can be found e.g. in "Hauptgruppe 10 'AntibiotikalAntiinfektiva'" of the Rote Liste.
Examples of anti-infectives can be found e.g. in "Hauptgruppe 10 'Antibiotika/Antiinfektiva' as well as "Hauptgruppe 21 'Antimykotika"' of the Rote Liste.
Examples of pharmaceutically active agents against dermatoses can be found e.g. in "Hauptgruppe 32 'Dermatika"' of the Rote Liste.
Examples of pharmaceutically active agents for wound healing can be found e.g. in "Hauptgruppe 32 'Dermatika'" as well as "Hauptgruppe 85 'Wund- und Narbenbehandlungsmittel'" of the Rote Liste.
The person skilled in the art will be able to select the appropriate pharmaceutically active agent according to the information presented therein and his general knowledge in the field.
Depending on the specific pharmaceutically active agent, the person skilled in the art will be able to adapt the specific composition accordingly either by obvious preliminary tests or by the general knowledge in the field.

As disclosed in WO-2005/115403 the expression of genes encoding antimicrobial peptides is also increased by Vitamin D3 and certain analogs thereof (e.g. lexacalcitol, seocalcitol, Vitamin D3 analog I). Thus, in one embodiment, Vitamin D3 an the analogs thereof which are being disclosed in said document are also included in the composition according of the invention.

In a preferred embodiment such further pharmaceutically active agent is selected from the group comprising antibiotics, anti-infectives, pharmaceutically active agents against dermatoses and active agents for wound healing.

Preferably, the effect of supporting of the innate immune system is essentially only mediated by urea.

As described herein, compositions comprising urea are able to support the innate immune system by increasing the expression of genes encoding antimicrobial peptides.
Optionally, this supporting effect of the compositions comprising urea can be complemented including antimicrobial peptides or variants thereof in the composition comprising urea.

Thus, a further embodiment of the present invention relates to compositions comprising urea and additionally comprising and antimicrobial peptide selected from the group containing defensins, cathelicidins and psoriasins or variants of any of said peptides.

The term "variants" as used according to the present invention and as far as this term relates to antimicrobial peptides includes homologues and/or orthologs and/or chemically modified peptides.

Preferred is a peptide comprising an amino acid sequence according to a sequence described by any of the GenBank Accession numbers listed herein, or a homologue and/or orthologue and/or chemically modified variant thereof.

The term "homologue" relates to peptides which are at least 70%, preferably 80%, more preferably 90%, most preferably 95% identical to a peptide according to any of the GenBank Accession numbers as listed herein. Preferably the degree of homology is calculated using the algorithm of Smith & Waterman (J. Mol. Biol. 147: 195-8) using a BLOSUM62-matrics and the value of 11.0 for inserting a gap and 1.0 for extending a gap.

The term "ortholog" (or "species homolog") denotes a polypeptide or protein obtained from one species that has homology to an analogous polypeptide or protein from a different species.
The algorithm for the detection of orthologue gene pairs from humans and mammalians or other species uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given, e.g-, in Nature, 2001, 409:860-921.

In the context of the present invention, a "chemically modified variant" shall mean a peptide, wherein the amino acids of the sequence have been modified to include additional chemical moieties.

Modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the [alpha]-amino groups of lysine, arginine, and histidine side chains ( T.E. Creighton, Proteins: Structure and Molecular Properties, W.H. Freeman & Co., San Francisco, pp. 79-86 (1983)), acetylation of the N-terminal amine, and amidation of any C-terminal carboxyl group.

Modifications further include alterations in the the native glycosylation pattern of the peptide. Modifications further include changes in the pattern of fatty acids coupled to the peptide, including changes in the pattern of myristylation, palmitylation, farnesylation. Modifications further include addition of tags or coupling to other moieties such as peptides or proteins. Modifications further include the insertion of a non-natural amino acid.

A number of variants of antimicrobial peptides have been described. Peptides which have been commercially developed have been described e.g in the following publications: Hancock et al. (2002), Curr Drug Targets Infect Disord, Vol. 2, pp. 79-83; Hancock et al. (1998) Trends Biotechnol, Vol. 16, pp. 82-88; Zasloff, M. (2002) N Engl J Med, Vol. 347, pp. 1199-1200. Peptides having been described in said publications and show an antimicrobial activity are incorporated by reference herein.

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### Example

### Compositions

### Emulsions were used as listed thereafter.

### Emulsion I

| COMPOUND/MIXTURE | | % (weight/weight) |
|---|---|---|
| | - Urea | 10% |
| Lipophilic emollient mixture: | | 13,4% |
| | - Isopropyl Myristate | |
| | - Paraffinum Liquidum | |
| | - Cetearyl Ethylhexanoate | |
| | - Dimethicone | |
| | - Persea Gratissima (Avocado) Oil | |
| Emulsifying mixture: | | 7.7% |
| | - Glyceryl Stearate | |
| | - PEG-40 Stearate | |
| | - Cetyl Alcohol | |
| | - Stearic Acid | |
| | - Sodium Carbomer | |
| Humectant/skin conditioning agent/pH adjuster mixture: | | 4,205% |
| | - Sorbitol | |
| | - Allantoin | |
| | - Lactic Acid | |
| Preservative mixture: | | 0,27% |
| | - Propylparaben | |
| | - Methylparaben | |
| | - 2-Bromo-2-Nitropropane-1,3-diol | |
| Fragance (Parfum): | | 0,1% |
| + Water | | up to 100% |

### Emulsion II

| COMPOUND/MIXTURE | | % (weight/weight) |
|---|---|---|
| | - Urea | 20% |
| Lipophilic emollient mixture: | | 13,4% |
| | - Isopropyl Myristate | |
| | - Paraffinum Liquidum | |
| | - Cetearyl Ethylhexanoate | |
| | - Dimethicone | |
| | - Persea Gratissima (Avocado) Oil | |
| Emulsifying mixture: | | 10,3% |
| | - Glyceryl Stearate | |
| | - PEG-40 Stearate | |
| | - Cetyl Alcohol | |
| | - Stearic Acid | |
| | - Sodium Carbomer | |
| | - Acrylates/C10-30 Alkyl Acrylate Crosspolymer | |
| Humectant/skin conditioning agent/pH adjuster mixture: | | 4,206% |
| | - Sorbitol | |
| | - Allantoin | |
| | - Lactic Acid | |
| Preservative mixture: | | 0,27% |
| | - Propylparaben | |
| | - Methylparaben | |
| | - 2-Bromo-2-Nitropropane-1,3-diol | |
| Fragance (Parfum): | | 0,1% |
| + Water | | up to 100% |

### Emulsion III

| COMPOUND/MIXTURE | | % (weight/weight) |
|---|---|---|
| | - Urea | --- |
| Lipophilic emollient mixture: | | 13,4% |
| | - Isopropyl Myristate | |
| | - Paraffinum Liquidum | |
| | - Cetearyl Ethylhexanoate | |
| | - Dimethicone | |
| | - Persea Gratissima (Avocado) Oil | |
| Emulsifying mixture: | | 9,0% |
| | - Glyceryl Stearate | |
| | - PEG-40 Stearate | |
| | - Cetyl Alcohol | |
| | - Stearic Acid | |
| | - Sodium Carbomer | |
| | - Acrylates/C10-30 Alkyl Acrylate Crosspolymer | |
| Humectant/skin conditioning agent/pH adjuster mixture: | | 4,205% |
| | - Sorbitol | |
| | - Allantoin | |
| | - Lactic Acid | |
| Preservative mixture: | | 0,27% |
| | - Propylparaben | |
| | - Methylparaben | |
| | - 2-Bromo-2-Nitropropane-1,3-diol | |
| Fragance (Parfum): | | 0,1% |
| + Water | | up to 100% |

### Study

### 1. Cell Culture

Primary human epidermal keratinocytes were prepared from neonatal foreskin as described previously (Grether-Beck et al, 1996, 2000, 2003, 2005, 2008) and maintained in culture under serum free conditions using a defined keratinocyte growth medium Keratinocyte SFM (Invitrogen, Heidelberg, Germany) supplemented with bovine pituitary extract (Invitrogen, Heidelberg, Germany) and recombinant epidermal growth factor (Invitrogen, Heidelberg, Germany). Cells were propagated up to passage 2 or 3 at 37°C and 5 % CO2 (Grether-Beck et al, 1996, 2000, 2003, 2005, 2008). For induction of differentiation NHEKs were seeded in 6well plates and grown up to confluence.

### 2. Treatment of human volunteers

Tests were conducted with human volunteers. The age ranged from 21 to 59 years. All were non-smokers and had no history of any severe skin disease.
The emulsions I, II, respectively III were applied to the skin of the volunteers as following:
All volunteers were treated once daily for 4 weeks with each of the the emulsions I, II and III at three different areas of buttock skin. Skin biopsies from the three areas were taken thereafter to test the gene expression as described thereafter.

### 3. Assessment of gene expression

For assessment of gene expression total RNA was extracted from frozen biopsies (Ø 4 mm) and gene expression measured by semiquantitative RT-PCR. For isolation of RNA from frozen skin biopsies, 600 µl lysis buffer from PeqGold Total RNA Kit (PeqLab, Erlangen, Germany) were added and the samples were disrupted in a MixerMill MM300 (Retsch, Haan, Germany) three times for 3 min with 30 Hz. 50 ng total RNA were used for cDNA synthesis. PCR reactions were performed in an Opticon 1 (MJ Research, Waltham, MA, USA) using Sybr QPCR Supermix w. Rox (Invitrogen, Karlsruhe, Germany). PCR conditions were as follows: activation of hot start taq polymerase 94° C 15 minutes; denaturation 95° C, 20 seconds; annealing, 55° C, 20 seconds; extension, 72° C 30 seconds. Each sample was subjected to PCR in double using the appropriate primer pairs for 45-50 cycles. For comparison of relative expression in real time PCR control cells and treated cells the 2 (-della delta C(T)) method was used (Livak KJ, and Schmittgen TD. Analysis of relative gene expression data using real time quantitative PCR and the 2 (-delta delta C(T)) method. Methods 2001: 25: 402 - 408). mRNA expression of the genes of interest was shown as fold induction as compared to untreated skin samples of the same volunteer.
Primers used are listed in Table 1.

**Table 1: Primer pairs for real time PCR**

| Gene | Sequence | Reference |
|---|---|---|
| 18S rRNA | 5´-GCCGCTAGAGGTGAAATTCTTG-3´ 5´-CATTCTTGGCAAATGCTTTCG´-3´ | |
| β-Defensin-1 (HBD-1) | 5´- TCAGCAGTGGAGGGCAATG -3´ 5´- CCTCTGTAACAGGTGCCTTGAAT -3´ | Liu et al. (1997) Genomics 43: 316-320 |
| β-Defensin-2 (HBD-2) | 5′- TTCCTGATGCCTCTTCCAGTTT -3′ 5′- GGATGACATATGGCTCCACTCTT -3′ | Diamond et al. (2000) Infect Immun 68: 113-119 |
| β-Defensin-3 (HBD-3) | 5´- TCAGCTGCCTTCCAAAGGA -3´ 5´- TTCTTCGGCAGCATTTTCG -3´ | Harder et al. (2001) J Biol Chem 276: 5707-13 |
| LL-37 | 5′-TCACCAGAGGATTGTGACTTCAA-3′ 5′-TGAGGGTCACTGTCCCCATAC-3′ | Agerberth et al. (1995) Proc Natl Acad Sci USA 92: 195-199 |
| Humpsor | 5′-TGCTGACGATGATGAAGGAGAA-3′ 5′-GCGAGGTAATTTGTGCCCTTT-3′ | Madsen et al. (1991) J Invest Dermatol 97: 701-712 |

For determination of relative gene expression in real time PCT the 2(-delta delta C(T))-method was used as described in Livak and Schmittgen (2001) "Analysis of relative gene expression data using real time quantitative PCR and the 2 (-delta delta C(T))-method" Methods 25: 401-408.
For statistical analysis, the experimental data were analyzed using the Wilcoxon signed-rank test.

### 4. Assessment of increase in amount of antimicrobial peptides.

The increase of the amount of antimicrobial peptides can be determined by using different methods.
Exemplary methods for the detection of β-defensin 2 are described e.g. in: Schmid et al, J Invest Dermatol 116:471-472, 2001; and Liu et al, J Invest Dermatol 118: 275-281,2002.
Exemplary methods for the detection of LL-37 are described e.g. in: Heilborn et al, J Invest Dermatol 120: 379-389, 2003; and Braff et al, J Invest Dermatol 124: 394-400, 2005

### 5. Results

### a) Cell Culture

Figure 1 shows results obtained in primary (subconfluent) human keratinocytes (HNKs) which have been cultured in serum free media as described earlier (1). HNKs were stimulated with 10mM urea for the indicated time points (3h, 6h, 12h, 24h) and processed for RNA analysis (1). Data are the result of a set of three samples for the control and two samples in parallel for the urea stimulated ones.
Table 2 indicates the relative gene expression levels after stimulation with urea as compared to untreated contol cells.
Figure 2 shows expression levels of HBD-2 and LL-37 in confluent human keratinocytes at different time points after treatment with different concentrations of urea.
Table 3 indicates the data depicted in Figure 2.

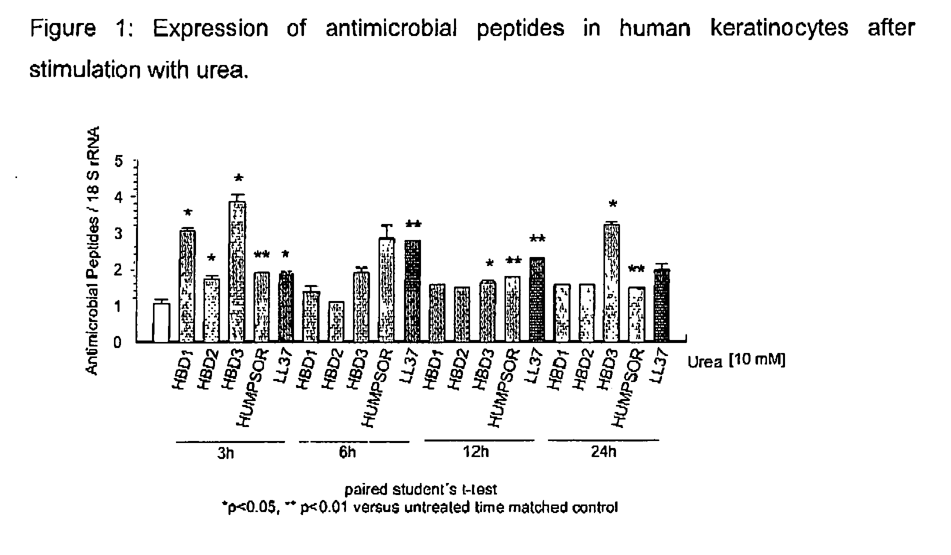

**Table 2: Expression of antimicrobial peptides in human keratinocytes after stimulation with urea at different time points after induction.**

| Time point | HBD1 | HBD2 | HBD3 | Humpsor | LL-37 |
|---|---|---|---|---|---|
| 3h | 3.100 | 1.8000 | 4.0000 | 1.9000 | 1.8000 |
| 3h | 3.000 | 1.7000 | 3.7000 | 1.9000 | 1.9000 |
| 6h | 1.3000 | 1.1000 | 2.0000 | 2.6000 | 2.8000 |
| 6h | 1.5000 | 1.1000 | 1.8000 | 3.1000 | 2.8000 |
| 12h | 1.6000 | 1.5000 | 1.6000 | 18000 | 2.3000 |
| 12h | 1.6000 | 1.5000 | 1.7000 | 18000 | 2.3000 |
| 24h | 1.6000 | 1.6000 | 3.2000 | 1.5000 | 2.1000 |
| 24h | 1.6000 | 1.6000 | 3.3000 | 1.5000 | 1.9000 |

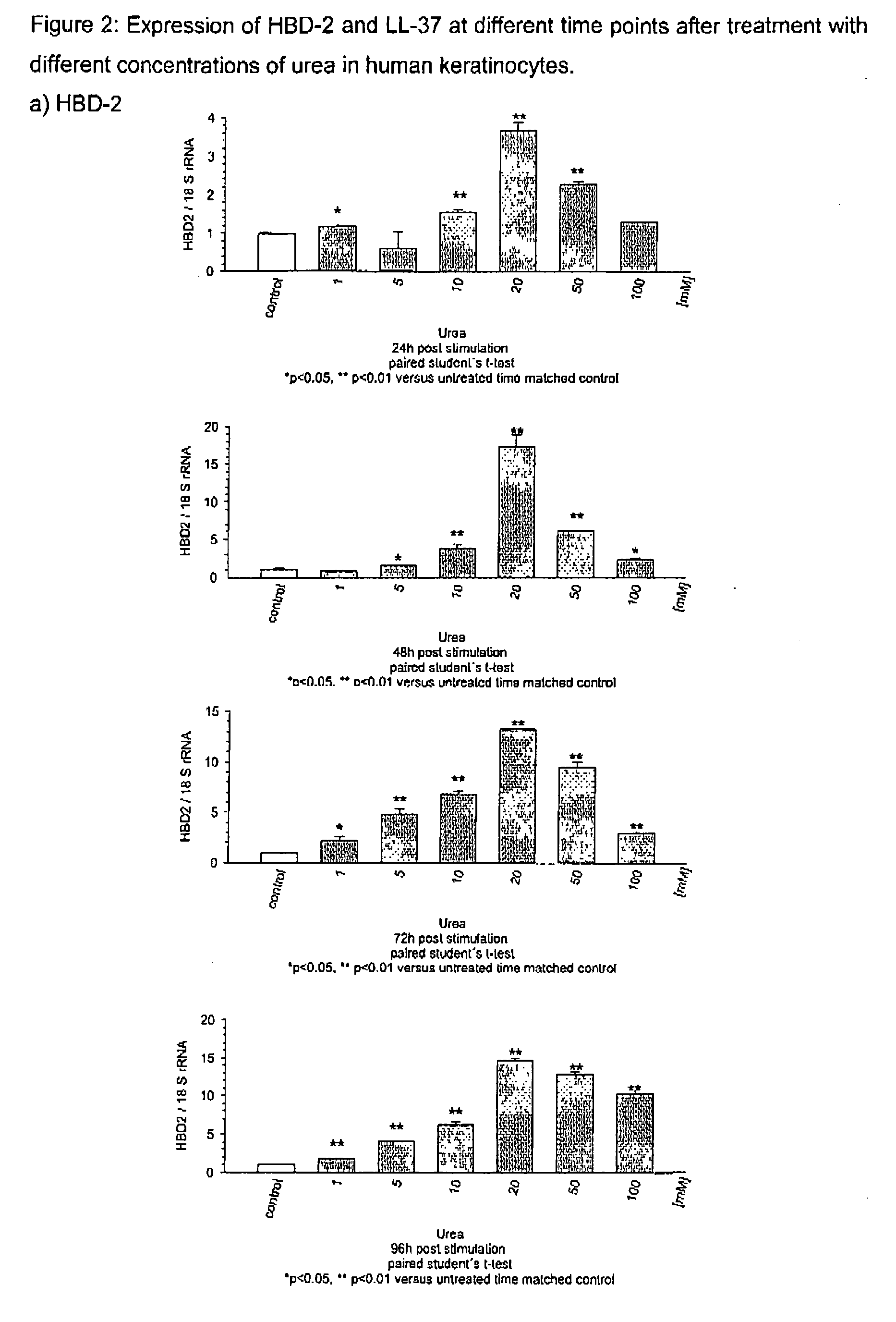

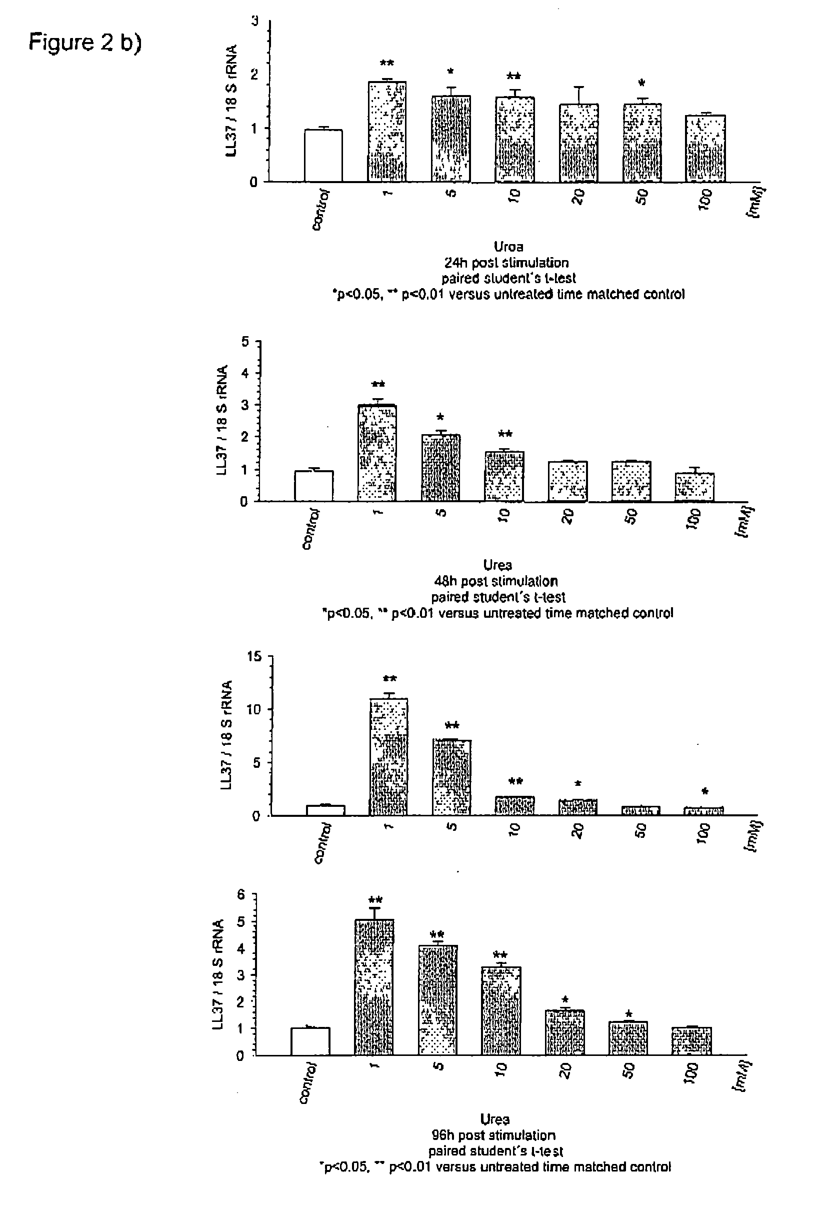

| |
|---|
| Table 3: Expression of HBD-2 and LL-37 at different time points after treatment with different concentrations of urea in human keratinocytes. |

### a) HBD-2

HBD2 24h

| 0mM urea | 1mM urea | 5mM urea | 10mM urea | 20mM urea | 50mM urea | 100mM urea |
|---|---|---|---|---|---|---|
| 1.0000 | 1.2000 | 0.8000 | 1.5000 | 3.9000 | 2.3000 | 1.3000 |
| 1.0000 | 1.2000 | 0.1000 | 1.5000 | 3.6000 | 2.3000 | 1.3000 |
| 1.0000 | 1.1000 | 0.9000 | 1.6000 | 3.5000 | 2.2000 | 1.3000 |

HBD2 48h

| 0mM urea | 1mM urea | 5mM urea | 10mM urea | 20mM urea | 50mM urea | 100mM urea |
|---|---|---|---|---|---|---|
| 1.0000 | 0.9000 | 1.7000 | 3.3000 | 19.1000 | 6.3000 | 2.3000 |
| 1.2000 | 0.9000 | 1.5000 | 4.4000 | 17.0000 | 6.2000 | 2.2000 |
| 1.1000 | 0.7000 | 1.6000 | 4.0000 | 16.2000 | 6.2000 | 2.6000 |

HBD2 72h

| 0mM urea | 1mM urea | 5mM urea | 10mM urea | 20mM urea | 50mM urea | 100mM urea |
|---|---|---|---|---|---|---|
| 1.0000 | 2.2000 | 5.4000 | 6.5000 | 13.3000 | 10.1000 | 2.9000 |
| 1.0000 | 2.6000 | 4.3000 | 7.0000 | 13.3000 | 9.3000 | 3.2000 |
| 0.9000 | 1.9000 | 4.9000 | 7.0000 | 13.2000 | 9.2000 | 2.8000 |

HBD2 96h

| 0mM urea | 1mM urea | 5mM urea | 10mM urea | 20mM urea | 50mM urea | 100mM urea |
|---|---|---|---|---|---|---|
| 1.0000 | 1.9000 | 4.1000 | 6.1000 | 15.0000 | 12.6000 | 10.2000 |
| 0.9000 | 1.7000 | 4.2000 | 5.9000 | 14.9000 | 12.7000 | 10.1000 |
| 1.1000 | 1.8000 | 4.2000 | 6.9000 | 14.2000 | 13.4000 | 10.9000 |

### Table 3 b)

LL-37 24h

| 0mM urea | 1mM urea | 5mM urea | 10mM urea | 20mM urea | 50mM urea | 100mM urea |
|---|---|---|---|---|---|---|
| 1.0000 | 1.8000 | 1.4000 | 1.6000 | 1.2000 | 1.3000 | 1.2000 |
| 0.9000 | 1.9000 | 1.7000 | 1.4000 | 1.3000 | 1.5000 | 1.3000 |
| 1.0000 | 1.9000 | 1.7000 | 1.7000 | 1.8000 | 1.5000 | 1.2000 |

LL-37 48h

| 0mM urea | 1mM urea | 5mM urea | 10mM urea | 20mM urea | 50mM urea | 100mM urea |
|---|---|---|---|---|---|---|
| 1.0000 | 3.2000 | 2.1000 | 1.5000 | 1.2000 | 1.2000 | 0.8000 |
| 0.9000 | 3.0000 | 2.2000 | 1.6000 | 1.3000 | 1.3000 | 1.1000 |
| 1.0000 | 2.8000 | 1.9000 | 1.6000 | 1.2000 | 1.2000 | 0.8000 |

LL-37 72h

| 0mM urea | 1mM urea | 5mM urea | 10mM urea | 20mM urea | 50mM urea | 100mM urea |
|---|---|---|---|---|---|---|
| 1.0000 | 11.2000 | 7.2000 | 1.8000 | 1.6000 | 0.8000 | 0.8000 |
| 0.9000 | 10.3000 | 7.1000 | 1.7000 | 1.4000 | 0.9000 | 0.8000 |
| 1.0000 | 11.4000 | 7.2000 | 1.7300 | 1.4000 | 0.9000 | 0.8000 |

LL37 96h

| 0mM urea | 1mM urea | 5mM urea | 10mM urea | 20mM urea | 50mM urea | 100mM urea |
|---|---|---|---|---|---|---|
| 1.0000 | 5.0000 | 3.9000 | 3.2000 | 1.6000 | 1.3000 | 1.1000 |
| 1.1000 | 4.7000 | 4.2000 | 3.4000 | 1.6000 | 1.3000 | 1.1000 |
| 1.0000 | 5.5000 | 4.2000 | 3.4000 | 1.8000 | 1.2000 | 1.0000 |

### b) Human volunteers

As shown in Table 4, treatment with emulsion I as well as treatment with emulsion II resulted in a significant increase of expression of the gene encoding HBD-2 (Table 4 a) and LL-37 (Table 4 b) as compared to treatment with emulsion III and as compared to the control.

**Table 4: Level of induction of expression of gene encoding HDB-2 and LL-37 by topical application of emulsions I, II and III. a) HBD-2**

| LL-37 | | | | |
|---|---|---|---|---|
| Volunteer No | Emulsion I (10% Urea) | Emulsion II (20% Urea) | Emulsion III (Placebo) | Untreated Control |
| 1 | 1,2 | 1,3 | 0,9 | 1 |
| 2 | 1,1 | 1,2 | 1,7 | 1 |
| 3 | 1,6 | 0,1 | 0,2 | 1 |
| 4 | 1,6 | 1,4 | 1,7 | 1 |
| 5 | 4,0 | 7,2 | 0,9 | 1 |
| 6 | 0,5 | 0,6 | 0.2 | 1 |
| 7 | not determined | not determined | not determined | 1 |
| 8 | 0,7 | 0,8 | 1,8 | 1 |
| 9 | 6,8 | 1,9 | 1,0 | 1 |
| 10 | 21,4 | 2,7 | 2,5 | 1 |
| 11 | 2,5 | 6,5 | 4,1 | 1 |
| 12 | 2,7 | 0,1 | 2,5 | 1 |
| 13 | 17,9 | 1,6 | 6,9 | 1 |
| 14 | 2,3 | 2,1 | 1,6 | 1 |
| 15 | 1,0 | 1,5 | 1,0 | 1 |
| 16 | 1,4 | 0,8 | 1,6 | 1 |
| 17 | 2,8 | 1,1 | 1,7 | 1 |
| 18 | 3,8 | 1,6 | 1,7 | 1 |
| 19 | 4,1 | 1,2 | 1,5 | 1 |
| 20 | 3,2 | 1,5 | 1,5 | 1 |
| 21 | 1,6 | 4,1 | 7,4 | 1 |

4b)

| β-Defensin-2 | | | | |
|---|---|---|---|---|
| Volunteer No | Emulsion I (10% Urea) | Emulsion II (20% Urea) | Emulsion III (Placebo) | Untreated Control |
| 1 | 2,6 | 3,6 | 1,3 | 1 |
| 2 | 1,7 | 1,2 | 1,1 | 1 |
| 3 | 3,3 | 1,7 | 1,0 | 1 |
| 4 | 1,2 | 2,7 | 1,1 | 1 |
| 5 | 3,4 | 3,2 | 0,5 | 1 |
| 6 | 1,9 | 3,0 | 0,4 | 1 |
| 7 | not determined | not determined | not determined | 1 |
| 8 | 1,8 | 1,7 | 1,2 | 1 |
| 9 | 13,4 | 2,1 | 1,1 | 1 |
| 10 | 2,3 | 5,0 | 1,5 | 1 |
| 11 | 7,6 | 3,3 | 2,4 | 1 |
| 12 | 6,2 | 3,1 | 1,7 | 1 |
| 13 | 28,1 | 4,8 | 18,4 | 1 |
| 14 | 1,8 | 1,3 | 0,9 | 1 |
| 15 | 1,3 | 2,0 | 1,4 | 1 |
| 16 | 1,3 | 0,8 | 0,5 | 1 |
| 17 | 1,6 | 1,0 | 0,8 | 1 |
| 18 | 1,8 | 0,9 | 1,1 | 1 |
| 19 | 3,9 | 1,6 | 1,6 | 1 |
| 20 | 3,4 | 2,6 | 1,3 | 1 |
| 21 | 7,2 | 6,0 | 1,8 | 1 |

Figure 3 depicts the relative expression rates of HBD-2 and LL-37 according to the data listed in Table 4

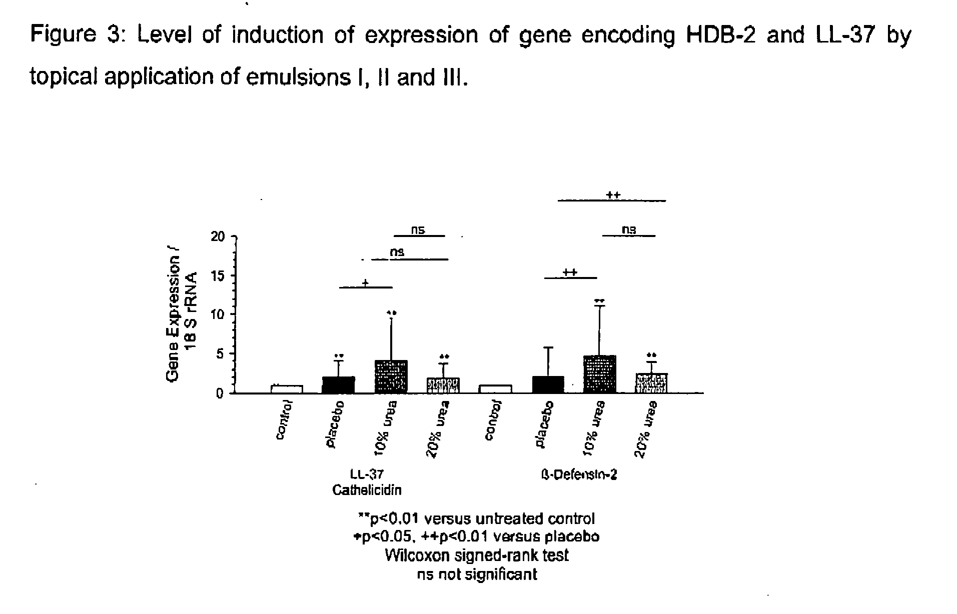

A statistical analysis of the data listed in Table 4 and illustrated in Figure 3 is given in Table 5.

**Table 5: Statistical analysis of level of induction of expression of gene encoding HDB-2 and LL-37 by topical application of emulsions I, II and III.**

| a) HBD-2 | | | | |
|---|---|---|---|---|
| | HBD-2 | | | |
| | control | Emulsion III (placebo) | Emulsion I (10%urea) | Emulsion II (20%urea) |
| Mean | 1.0000 | 2.0550 | 4.7900 | 2.5800 |
| median | 1.0000 | 1.1500 | 2.4500 | 2.3500 |
| SD | 0.0000 | 3.8766 | 6.2543 | 1.4508 |
| SE | 0.0000 | 0.8668 | 1.3985 | 0.3244 |
| 95%conf | 0.0000 | 1.8143 | 2.9271 | 0.6790 |
| 99%conf | 0.0000 | 2.4801 | 4.0013 | 0.9282 |
| size | 20.0000 | 20.0000 | 20.0000 | 20.0000 |
| total | 20.0000 | 41.1000 | 95.8000 | 51.6000 |
| min | 1.0000 | 0.4000 | 1.2000 | 0.8000 |
| max | 1.0000 | 18.4000 | 28.1000 | 6.0000 |
| min | 1.0000 | 0.4000 | 1.2000 | 0.8000 |

**Table 5b)**

| | LL-37 | | | |
|---|---|---|---|---|
| | control | Emulsion III (placebo) | Emulsion I (10%urea) | Emulsion II (20%urea) |
| Mean | 1.0000 | 2.1200 | 4.1100 | 1.9650 |
| median | 1.0000 | 1.6500 | 2.4000 | 1.4500 |
| SD | 0.0000 | 1.9182 | 5.5486 | 1.8919 |
| SE | 0.0000 | 0.4289 | 1.2407 | 0.4230 |
| 95%conf | 0.0000 | 0.8978 | 2.5969 | 0.8854 |
| 99%conf | 0.0000 | 1.2272 | 3.5499 | 1.2104 |
| size | 20.0000 | 20.0000 | 20.0000 | 20.0000 |
| total | 20.0000 | 42.4000 | 82.2000 | 39.3000 |
| min | 1.0000 | 0.2000 | 0.5000 | 0.1000 |
| max | 1.0000 | 7.4000 | 21.4000 | 7.2000 |
| min | 1.0000 | 0.2000 | 0.5000 | 0.1000 |

## Claims

1. A use of a composition comprising urea for increasing the expression of at least one gene encoding an antimicrobial peptide.

2. A use according to claim 1 wherein said gene is a gene encoding a peptide selected from the group comprising of defensins, cathelicidins and psoriasins.

3. A use according to claim 1 or 2, wherein the expression of any of said gene is increased by a factor of at least 2, preferably of at least 3, more preferably of at least 5, even more preferably of at least 10, most preferably of at least 25.

4. A use according to any one of claims 1 to 3 wherein said composition comprises 1 to 30% by weight, based on the total amount of the composition, of urea.

5. A use according to any one of claims 1 to 4 for preventing and/or treating of a disease in which microorganisms have an influence.

6. A use according to claim 5 in which said disease is caused by microorganisms.

7. A use according to claim 5 or 6 wherein said disease is a disease affecting the epidermis.

8. A use according to any one of claim 5 or 7 wherein said disease is a skin disease.

9. A use according to any one of claims 1 to 8 wherein said composition is applied topically onto the skin, administered by injection or by a depot, or administered orally.

10. A use according to claim 9 wherein said composition is applied at least once a day for at least 2 weeks topically and/or administered by injection and optionally longer until the expression of any of said genes is increased by a factor of at least 2, preferably at least 3, more preferably at least 5, even more preferably of at least 10, most preferably of at least 25.

11. A use according to claim 9 or 10 wherein the treated tissue, preferably skin, is normal and/or already damaged tissue, preferably skin, of a mammal, preferable of a human being.

12. A use according to claim 11 wherein said damages are wounds.

13. A use according to any one of claims 1 to 12 wherein said composition is a pharmaceutical composition only comprising urea as a pharmaceutically active agent.

14. A use according to any one of claims 1 to 12 wherein said composition is a pharmaceutical composition comprising a further pharmaceutically active agent.

15. A use according to claim 14 wherein said further pharmaceutically active agent is selected from the group comprising antibiotics, anti-infectives, pharmaceutically active agents against dermatoses and for wound healing.

16. A composition according to claim 13 or 14 comprising at least one antimicrobial peptide, preferably selected from the group comprising defensins, psoriasins and cathelicidins or variants of any of said peptides.
